# EUROPÄISCHE PATENTSCHRIFT

(11) **EP 1 420 757 B1**
(45) Veröffentlichungstag und Bekanntmachung des Hinweises auf die Patenterteilung: **01.03.2006**
(21) Anmeldenummer: 02767375.5
(22) Anmeldetag: 13.08.2002
(51) Int. Cl.: A61K 8/06, A61K 8/49, A61K 31/505, A61Q 19/00

(54) **Kosmetische Mikroemulsionen enthaltend Ectoin oder Derivate**
Cosmetic microemulsions comprising ectoine or derivatives
Microémulsion cosmétique comprenant ectoine ou dérivés

(30) Priorität: 01.09.2001 DE 10142693
(43) Veröffentlichungstag der Anmeldung: 26.05.2004
(73) Patentinhaber: Sebapharma GmbH & Co., 56136 Boppard-Bad Salzig (DE)
(72) Erfinder: GOTTFREUND, Joachim, 56154 Boppard (DE)
(74) Vertreter: Lippert, Hans-Joachim
(86) Internationale Anmeldenummer: PCT/EP2002/009056
(87) Internationale Veröffentlichungsnummer: WO 2003/020237

(56) Entgegenhaltungen:
- EP-A- 0 328 806
- DE-A- 4 342 560
- DE-A- 19 933 460
- DE-A- 19 933 461
- DE-A- 19 933 462
- DE-A- 19 933 466
- DATABASE BIOSIS [Online] BIOSCIENCES INFORMATION SERVICE, PHILADELPHIA, PA, US; 2000 ANONYMOUS: "Final report on the safety assessment of PEG-20 Sorbitan Cocoate;PEG-40 Sorbitan Diisostearate; PEG-2, -5, and -20 Sorbitan Isostearate; PEG-40 and -75 Sorbitan Lanolate; PEG-10, -40, -44, -75, and -80 Sorbitan Laurate; PEG-3, and -6 Sorbitan Oleate; PEG-80 Sorbitan Palmitate; PEG-40 Sorbitan Periso" Database accession no. PREV200100083530 XP002233306 & INTERNATIONAL JOURNAL OF TOXICOLOGY, Bd. 19, Nr. Supplement 2, 2000, Seiten 43-89, ISSN: 1091-5818

## Beschreibung

Gegenstand der Erfindung ist eine kosmetische, optisch transparente, stabile Zubereitung enthaltend Ectoin oder Ectoinderivate.

Es ist bekannt, dass (S)-1,4,5,6-Tetrahydro-2-methyl-4-pyrimidincarbon-Säure (Ectoin) in extrem halophilen Mikroorganismen vorkommt und bei der Osmoseregulation dieser Organismen eine Rolle spielt (E.A. Galinski et al., Eur. J. Biochem., 149 (1985), Seite 135-139). Es ist deshalb bereits in der DE 43 42 560 A1 beschrieben worden, dass Ectoin und Ectoinderivate als Feuchtigkeitsspender verwendet werden können.

Die Verwendung von Ectoin und Ectoinderivaten in Emulsionen als Haut- und Zellschutz ist bekannt (J. Bünger et al. "Ectoin - A new class of ingredient for skin protection", Merck KGaA, CHN Cosmetics, D-64271 Darmstadt, Germany). Ectoin und seine Derivate sind Substanzen, die mit der lebenden Zelle in Kontakt treten müssen und dort z.B. mit DNA einen Komplex bilden, der diese gegen UV-Strahlung schützt. Die Wirksamkeit von Ectoin und Ectoinderivaten ist also nur gegeben, wenn sie in Haut penetrieren können.

In den DE 199 33 460, DE 199 33 461, DE 199 33 462 und DE 199 33 466 sind spezielle Zubereitungen und Kombination von Ectoin und Ectoinderivaten beschrieben, wobei auch erwähnt wird, diese Wirkstoffe in Form von Mikroemulsionen zur Verfügung zu stellen. In den Beispielen sind jedoch nur Dispersionen und Emulsionen beschrieben mit relativ hohen Mengen an Ectoin oder Hydroxyectoin.

In diesen herkömmlichen Galeniken, die O/W bzw. W/O-Systeme darstellen, ist die Freisetzung aus der galenischen Matrix und die Diffusion des Ectoins oder der Ectoinderivate nach eigenen Untersuchungen gering (Prof. Daniels, TU Braunschweig "Untersuchungen zur Permeation von Ectoin durch exzidiertes humanes Stratum corneum"). Daher müssen hohe Konzentrationen an Ectoin und seinen Derivaten in den Emulsionen eingesetzt werden, die meist bei 1%, bezogen auf die Gesamtformulierung, liegen. Aber selbst bei diesen hohen Ectoinkonzentrationen ist eine Diffusion durch die Haut nur reduziert möglich (eigene Untersuchungen).

Zur Verbesserung der Wirksamkeit sind im pharmazeutischen Bereich transdermale Systeme im Einsatz, die die Wirkstofffreisetzung und ihre Diffusion begünstigen (Morganti et al., Cosmetics & Toiletries, Vol. 114, No. 2, February 1999, Seite 53 - 58, "The Transdermal Cosmetic Delivery System"). Diese bauen jedoch auf einer Polyacrylatmatrix auf (Feststoff oder Gel), aus der der Wirkstoff freigesetzt wird. Dadurch ist ein Einsatz nur lokal möglich. Auch Mikroemulsionen führen zu sehr unterschiedlichen Ergebnissen in Abhängigkeit vom Wirkstoff und den sonstigen Komponenten; vgl. C. Christiansen, "Mikroemulsionen zur Penetrationsverbesserung", Dissertation Universität Kiel, 2001.

Die Erfindung hat sich die Aufgabe gestellt, kosmetische Zubereitungen von Ectoin und Ectoinderivaten zur Verfügung zu stellen, welche hoch wirksam sind, stabil und optisch transparent.

Diese Aufgabe wird gelöst durch die kosmetische Zubereitung gemäß Anspruch 1, d.h. kosmetische, optisch transparente, stabile Zubereitungen enthaltend:
a) 0,05 bis 1 Gew.-% eines Ectoins oder Ectoinderivats der allgemeinen Formel 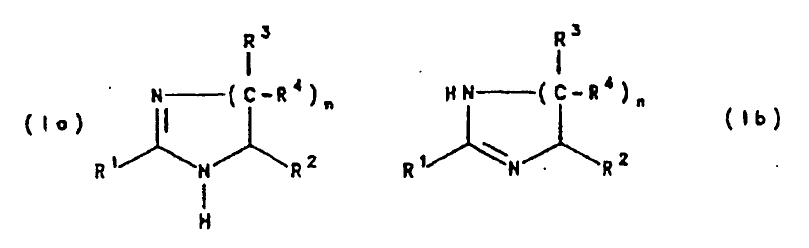 in der R¹ ein Wasserstoff oder eine Alkylgruppe mit 1 bis 4 Kohlenstoffatomen ist,
   R² H, -COOH, -COO-Alkyl mit 1 bis 4 Kohlenstoffatomen oder -CONH-R⁵ ist,
   wobei R⁵ H, eine Alkylgruppe mit 1 bis 4 Kohlenstoffatomen, eine Aminosäure, ein Dipeptid oder ein Tripeptid ist,
   R³ und R⁴ gleich oder verschieden sind und H oder eine OH-Gruppe sind und
   n eine Zahl von 1 bis 3 ist,
b) 4 bis 15 Gew.-% einer Ölkomponente,
c) 30 bis 50 Gew.-% eines oder mehrerer Cotenside
d) gegebenenfalls 0,1 bis 1 Gew.-% einer physiologisch erträglichen organischen Säure,
e) gegebenenfalls bis zu 10 Gew.-% Pflegestoffe und/oder Vitamine
f) 0,2 bis 1 Gew.-% Konservierungsmittel, sowie
g) 20 bis 60 Gew.-% demineralisiertes Wasser

Vorzugsweise werden eingesetzt Ectoine und Ectoinderivate, in denen R¹ = H oder eine Alkylgruppe mit 1 bis 4 Kohlenstoffatomen, R² = H oder COOH-Gruppe und n = 2 ist, wobei diese Wirkstoffe vorzugsweise in Mengen eingesetzt werden zwischen 0,1 und 0,5 Gew.-%.

Weiterhin ist der pH-Wert mit physiologisch unbedenklichen organischen Säuren wie Milchsäure oder Zitronensäure auf einen pH-Wert von 5,5 ± 0,5 eingestellt.

Schließlich können die Zubereitungen bis 10% zusätzliche Pflegestoffe und Vitamine enthalten wie Vitamin-A-Acetat, Retinol, Vitamin-E-Acetat, Tocopherol, Panthenol, Ceramide I-VI oder Gemische derselben. Schließlich enthalten die Zubereitungen 0,2 bis 1,0 Gew.-% Konservierungsmittel sowie 20 bis 60 Gew.-% demineralisiertes Wasser.

Als Ölkomponenten können eingesetzt werden: Mineralöl, natürliche Öle und Ester von linearen C₆-C₂₂-Fettsäuren mit linearen C₂-C₂₂-Fettalkoholen, Ester von verzweigten C₆-C₁₃-Carbonsäuren mit linearen C₆-C₂₂-Fettalkoholen, bevorzugt Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen, Triglyceride auf Basis C₆-C₁₀-Fettsäuren, flüssige Mono-/Di/Triglycerid-Mischungen auf Basis C₆-C₁₈-Fettsäuren.

Besonders bevorzugt sind Ethylcocoat, Hexyldecyllaurate und Ethylhexylstearate.

Als Cotenside können einzeln oder als Gemisch eingesetzt werden:

Mono-, Di-, Triglyceride von C₆-C₈ linearen Fettsäuren mit 6 bis 10 Mol Ethylenoxid, Mono-, Di- und Triester von C₁₅-C₂₀ linearen Fettsäuren mit Glycerinpolymeren, die zwischen 4 und 10 Glycerineinheiten enthalten, Mono-, Di- und Triester von C₁₀-C₁₅ linearen Fettsäuren mit Sorbitol, die zwischen 5 und 80 EO-. Einheiten enthalten können.

Ferner sind geeignet Sorbitanester wie Sorbitanmonoisostearat, Sorbitansesquiisostearat, Sorbitandiisostearat, Sorbitantriiosstearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmono-ricinoleat, Sorbitansesquiricinoleat, Sorbitandiricinoleat, Sorbitantriricinoleat, Sorbitanmonohydroxystearat, Sorbitansesquihydroxystearat, Sorbitandihydroxy-stearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technischen Gemische. Ebenfalls geeignet sind Anlagerungsprodukte von 1 bis 30, vorzugsweise 5 bis 80 Mol Ethylenoxid an die genannten Sorbitanester.

Besonders geeignet sind Gemische aus Polysorbat 20 mit Polyglyceryl-6 Dioleate oder PEG-8 Caprylic-capric Glyceride.

Als Konservierungsstoffe kommen in Frage Phenoxyethanol, Benzylalkohol, Ethyl-, Methyl-, Butyl-, Propyl-, Isobutylparabene, die genannten Parabene einzeln oder in Kombination.

Die erfindungsgemäßen kosmetischen Zubereitungen stellen eine Sonderform von Mikroemulsion dar, die sich in ihren Eigenschaften von herkömmlichen E-mulsionstypen, nämlich Öl in Wasser, Wasser in Öl oder Triple-Emulsionen unterscheidet. Es handelt sich vielmehr um eine Art Mikro-Emulsion, deren Teilchengröße überwiegend im Bereich zwischen 10 und 20 nm liegt und ein Gleichgewicht zwischen micellaren Strukturen und lamellaren einphasigen Strukturen aufweist. Diese Zubereitungen sind daher optisch transparent und isotop, wodurch sie sich optisch von herkömmlichen Cremes und Lotionen unterscheiden. Erstaunlicherweise sind diese Zubereitungen thermodynamisch stabil, zeigen keine Phasentrennung und sind somit praktisch unbegrenzt lagerfähig, während herkömmliche W/O- bzw. O/W-Emulsionen diese guten Eigenschaften nicht aufweisen. Von besonderer Bedeutung ist, dass aus diesen erfindungsgemäßen Zubereitungen die Wirkstoffe Ectoin und Ectoinderivate wesentlich besser und tiefer in die Haut eindringen und dort die Wirkung entfalten können, ohne dass es zu Hautreizungen kommt. Es ist deshalb möglich, den Gehalt an Wirkstoffen in diesen Zubereitungen sehr viel niedriger zu halten, als es in herkömmlichen Zubereitungen nötig war.

In den nachfolgenden Beispielen sind bevorzugte Zubereitungen beschrieben.

### Beispiel 1

| | Gew.-% |
|---|---|
| Polyglyceryl-6-Dioleate | 10 - 15 |
| Ehtylcocoate | 5-10 |
| PEG-8 Caprylic/Capric Glyceride | 10-15 |
| Polysorbate 20 | 20-25 |
| Pflegestoffe | 0,5-10 |
| Ectoin | 0,05 - 1 |
| organische Säure | 0,1-1 |
| Parfum | 0,5-5 |
| Konservierungsmittel | 0,2- 1 |
| Wasser | ad. 100 |
| pH-Wert: | 5,5 ± 0,5 |

### Beispiel 2

| | Gew.-% |
|---|---|
| Polyglyceryl-6-Dioleate | 10 - 15 |
| Hexyldecyllaurate | 5-10 |
| Hexyldecanol | 5-10 |
| PEG-8 Caprylic/Capric Glyceride | 10 - 15 |
| Polysorbate 20 | 20 - 25 |
| Pflegestoffe | 0,5 - 10 |
| Ectoin | 0,05 - 1 |
| organische Säure | 0,1 - 1 |
| Parfum | 0,5 - 5 |
| Konservierungsmittel | 0,2- 1 |
| Wasser | ad. 100 |
| pH-Wert: | 5,5 ± 0,5 |

### Beispiel 3

| | Gew.-% |
|---|---|
| Polyglyceryl-6-Dioleate | 10 - 15 |
| Ethylhexalstearate | 10-15 |
| PEG-8 Caprylic/Capric Glyceride | 10-15 |
| Polysorbate 20 | 20-25 |
| Pflegestoffe | 0,5-10 |
| Ectoin | 0,05 - 1 |
| organische Säure | 0,1 - 1 |
| Parfum | 0,5 - 5 |
| Konservierungsmittel | 0,2- 1 |
| Wasser | ad. 100 |
| pH-Wert: | 5,5 ± 0,5 |

## Patentansprüche

1. Kosmetische, optisch transparente, stabile Zubereitung enthaltend:
a) 0,05 bis 1 Gew.-% eines Ectoins oder Ectoinderivats der allgemeinen Formel 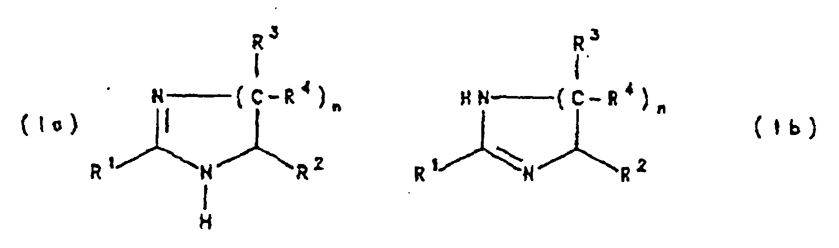 in der R¹ ein Wasserstoff oder eine Alkylgruppe mit 1 - 4 Kohlenstoffatomen ist,
R² H, -COOH, -COO-Alkyl mit 1 bis 4 Kohlenstoffatomen oder -CONH-R⁵ ist,
wobei R⁵ H, eine Alkylgruppe mit 1 bis 4 Kohlenstoffatomen, eine Aminosäure, ein Dipeptid oder ein Tripeptid ist,
R³ und R⁴ gleich oder verschieden sind und H oder eine OH-Gruppe sind und
n eine Zahl von 1 bis 3 ist,
b) 4 bis 15 Gew.-% einer Ölkomponente,
c) 30 bis 50 Gew.-% eines oder mehrerer Cotenside
d) gegebenenfalls 0,1 bis 1 Gew.-% einer physiologisch verträglichen organischen Säure,
e) gegebenenfalls bis zu 10 Gew.-% Pflegestoffe und/oder Vitamine
f) 0,2 bis 1 Gew.-% Konservierungsmittel, sowie
g) 20 bis 60 Gew.-% demineralisiertes Wasser

2. Kosmetische Zubereitung gemäß Anspruch 1, **dadurch gekennzeichnet, dass**
R¹ H oder Alkylgruppe mit 1 - 4 Kohlenstoffatomen,
R² H oder eine COOH-Gruppe und
n = 2 ist.

3. Kosmetische Zubereitung gemäß Anspruch 1 oder 2 **dadurch gekennzeichnet, dass** der Gehalt an Ectoin oder Ectoinderivat 0,1 bis 0,5 Gew.-% beträgt.

4. Kosmetische Zubereitung gemäß einem der Ansprüche 1 bis 3, **dadurch gekennzeichnet, dass** der pH-Wert mit organischen Säuren auf einen pH-Wert von 5,5 ± 0,5 eingestellt ist.

5. Kosmetische Zubereitung gemäß einem der Ansprüche 1 bis 4, **dadurch gekennzeichnet, dass** sie zusätzlich Tocopherol, Tocopherylacetat, Retinol, Retinylpalmitat, Retinylacetat, Panthenol, Ceramide I-VI sowie Gemische derselben enthalten.

## Claims

1. Cosmetic, optically transparent, stable preparation containing:
a) 0.05 to 1 % by wt. of an ectoine or ectoine derivative of the general formula 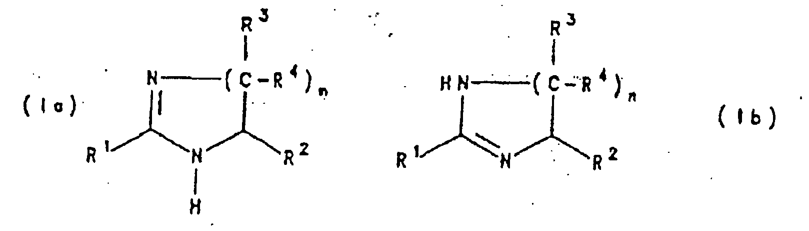 in which R¹ is a hydrogen or an alkyl group having 1 - 4 carbon atoms,
R² is H, -COOH, -COO alkyl having 1 to 4 carbon atoms or -CONH-R⁵,
R⁵ being H, an alkyl group having 1 to 4 carbon atoms, an amino acid, a dipeptide or a tripeptide,
R³ and R⁴ are identical or different and are H or an OH group, and
n is a number from 1 to 3,
b) 4 to 15 % by wt. of an oil component,
c) 30 to 50 % by wt. of one or more cosurfactants,
d) possibly 0.1 to 1 % by wt. of a physiologically compatible organic acid,
e) possibly up to 10 % by wt. curatives and/or vitamins
f) 0.5 to 1 % by wt. preservative, as well as
g) 20 to 60 % by wt. demineralised water.

2. Cosmetic preparation according to claim 1, **characterised in that**
R¹ is H or an alkyl group having 1 - 4 carbon atoms,
R² is H or a COOH group, and
n = 2.

3. Cosmetic preparation according to claim 1 or 2, **characterised in that** the content of ectoine or ectoine derivative is 0,1 to 0.5 % by wt.

4. Cosmetic preparation according to one of claims 1 to 3, **characterised in that** the pH value with organic acids is set to a pH value of 5.5 ± 0.5.

5. Cosmetic preparation according to one of claims 1 to 4, **characterised in that** it additionally contains tocopherol, tocopheryl acetate, retinol, retinyl palmitate, retinyl acetate, panthenol and ceramides I-VI as well as mixtures thereof.

## Revendications

1. Compositions cosmétique stable, optiquement transparente, contenant :
a) 0,05 à 1% en poids d'une ectoïne ou d'un dérivé d'ectoïne de la formule générale : 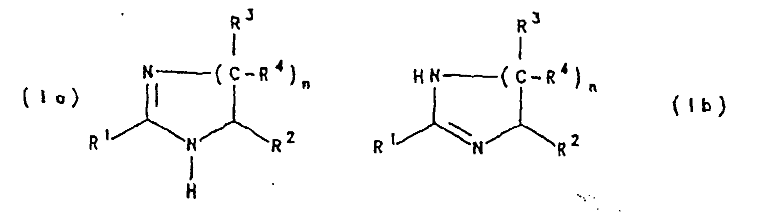
dans laquelle R¹ est hydrogène ou un groupe alkyle avec 1-4 atomes de carbone,
R² est H, -COOH, -COO-alkyle avec 1 à 4 atomes de carbone ou -CONH-R⁵, où R⁵ est H, un groupe alkyle avec 1-4 atomes de carbone, un acide aminé, un dipeptide ou un tripeptide,
R³ et R⁴ sont identiques ou différents et sont H ou un groupe OH, et
n est un nombre de 1 à 3,
b) 4 à 15% en poids d'un composant huileux,
c) 30 à 50% en poids d'un ou plusieurs co-tensioactifs,
d) le cas échéant, 0,1 à 1% en poids d'un acide organique physiologiquement compatible,
e) le cas échéant, jusqu'à 10% en poids de substances de soin et/ou de vitamines,
f) 0,5 à 1% en poids d'un agent de conservation, ainsi que
g) 20 à 60% en poids d'eau déminéralisée.

2. Composition cosmétique selon la revendication 1, **caractérisée en ce que**
R¹ est H ou un groupe alkyle avec 1-4 atomes de carbone,
R² est H ou un groupe COOH, et
n = 2.

3. Composition cosmétique selon la revendication 1 ou 2, **caractérisée en ce que** la teneur en ectoïne ou dérivé d'ectoïne se situe dans l'intervalle allant de 0,1 à 0,5% en poids.

4. Composition cosmétique selon l'une des revendications 1 à 3, **caractérisée en ce que** le pH est ajusté avec des acides organiques, à une valeur de 5,5 ± 0,5.

5. Composition cosmétique selon l'une des revendications 1 à 4, **caractérisée en ce qu'**elle contient en outre, le tocophérol, l'acétate de tocophéryle, le rétinol, le palmitate de rétinyle, le panthénol, les céramides I-VI ainsi que leurs mélanges.
